# DEMANDE DE BREVET EUROPEEN

(11) **EP 3 575 305 A1**
(43) Date de publication de la demande: **04.12.2019**
(21) Numéro de dépôt: 18175310.4
(22) Date de dépôt: 31.05.2018
(51) Int. Cl.: C07D 498/04

(54) **PROCÉDÉ DE FABRICATION DE COMPOSÉS COMPRENANT UN GROUPE FONCTIONNEL OXAZOLOPYRIDINONES**

(71) Demandeur: Biocidal Alternative Solutions, 5310 Eghezée (BE)
(72) Inventeur: HANS, Morgan, 4900 SPA (BE)
(74) Mandataire: Calysta NV

(57) **Abrégé**

La présente invention se rapporte à un procédé de fabrication de composés comprenant un groupe fonctionnel oxazolopyridinones par la réaction entre un composé comprenant un groupe caractéristique hydroxyaminopyridine de formule (1) et un composé comprenant un groupe caractéristique diarylcarbonate de formule (2). I :

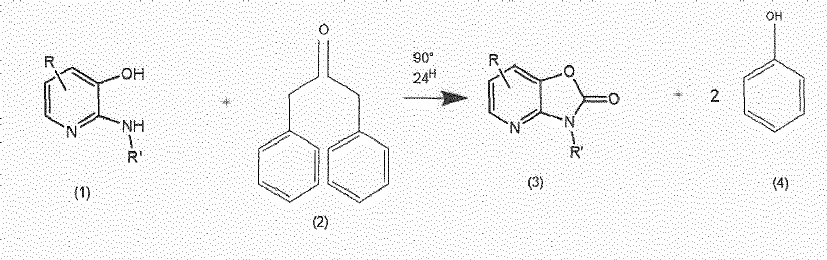

## Description

### Domaine technique

La présente invention se rapporte à un procédé de fabrication de composés comprenant un groupe fonctionnel oxazolopyridinone.

### Arrière-plan technologique de l'invention

Dans un premier aspect, la présente invention concerne donc un procédé de fabrication de composés comprenant un groupe fonctionnel oxazolopyridinone de formule (3) éventuellement substitué : 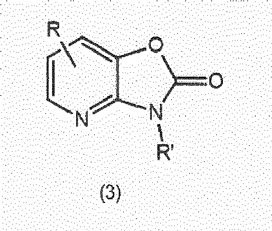 par une réaction entre un composé comprenant un groupe fonctionnel hydroxyaminopyridine de formule (1) éventuellement substitué : 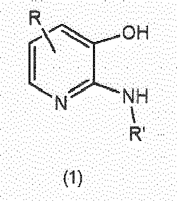 et un composé comprenant un groupe fonctionnel diarylcarbonate de formule (2) éventuellement substitué :

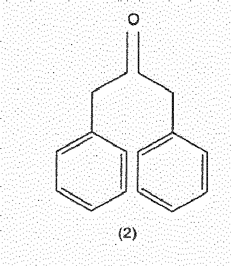

### Etat de la technique

Des procédés basés sur une réaction à partir d'une aminohydroxypyridine en présence d'une base et d'un agent de carbonylation dans un solvant sont connus comme par exemple des documents CN105924454 et WO2009130487.

Ces procédés font intervenir le triphosgène qui est un substitut plus sûr du phosgène. Cependant le triphosgène reste un produit toxique car il se décompose en phosgène par échauffement ou par réaction avec un groupe nucléophile. De telle sorte qu'il est nécessaire de prendre les mêmes précautions de sécurité pour faire intervenir le triphosgène que le phosgène. Le triphosgène est un solide et requiert par sa nature l'ajout de solvant qui laisse des traces dans le produit fini et qui demande des étapes de purification supplémentaires pour ne pas laisser de traces toxiques dans le produit fini.

On connaît du document US2012108809 l'utilisation de phosgène, triphosgène, carbonyldiimidazole ou diarylcarbonates comme réactifs donneurs de groupement carbonyl pour qu'ils réagissent avec des composés de type butyn-2-ol substitués par du chlore et du fluor dans le but d'obtenir des composés de type benzoxazine-2-one substitués par du chlore et du fluor pour la fabrication d'Efavirenz.

Si selon ce procédé les diarylcarbonates semblent appropriés, le procédé requiert la mise en solution d'un dérivé d'alcool aminé substitué par du chlore et du fluor dans un solvant aprotique tel que le tétrahydrofurane (THF) et en présence d'une base telle que le 1,8-diazabicyclo[5.4.0]undéc-7-ène (DBU) qui déprotone la molécule et la rend plus nucléophile donc plus apte à réagir avec le donneur de carbonyle, lequel est ajouté au mélange réactionnel.

Il apparaît toutefois un besoin de disposer d'un procédé permettant d'obtenir des oxazolopyridinones qui est plus propre, moins couteux, adaptable industriellement et plus rapide tout en restant flexible par rapport aux types de composés de départ.

### Résumé de l'invention :

L'invention a pour but de résoudre les problèmes mentionnés ci-avant en procurant un procédé de fabrication de composés comprenant un groupe fonctionnel oxazolopyridinone de formule (3) éventuellement substitué 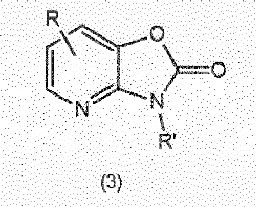 par une réaction entre un composé comprenant un groupe fonctionnel hydroxyaminopyridine de formule (1) éventuellement substitué : 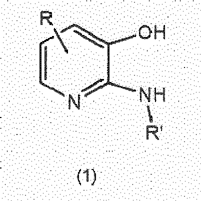 et un composé comprenant un groupe fonctionnel diarylcarbonate de formule (2) éventuellement substitué : 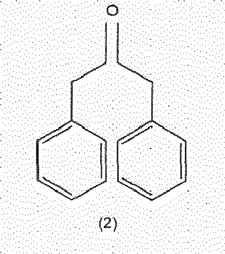 tel qu'indiqué au début, caractérisé en ce qu'il comporte une mise en suspension et une réaction dudit composé comprenant un groupe fonctionnel hydroxyaminopyridine de formule (1) dans ledit composé comprenant un groupe fonctionnel diarylcarbonate de formule (2) à un état fondu.

Contre toute attente, il a été observé selon l'invention qu'il était possible d'utiliser la combinaison de diarylcarbonates éventuellement substitués fondus qui sont d'une part beaucoup plus propres, moins toxiques pour l'environnement et pour la santé et d'autre part qui ne requièrent pas de solvants, ni de base.

Selon l'invention, le composé comprenant un groupe fonctionnel hydroxyaminopyridine éventuellement substitué est à un état solide lorsque le composé comprenant un groupe fonctionnel diarylcarbonate éventuellement substitué est à un état fondu. Il y a ainsi formation d'une suspension du composé comprenant un groupe fonctionnel hydroxyaminopyridine éventuellement substitué dans le composé comprenant un groupe fonctionnel diarylcarbonate éventuellement substitué évitant de cette manière la contamination du milieu réactionnel par des composés qui devront être éliminés ultérieurement.

Par conséquent, dans le procédé selon la présente invention, des composés comprenant un groupe fonctionnel oxazolopyridinone sont obtenus en réduisant le nombre de matières exogènes ajoutées.

Le procédé selon la présente invention est donc plus propre, moins couteux et particulièrement flexible et industrialisable, l'utilisation du phosgène ou du triphosgène qui sont des produits compliqués à manipuler et toxiques n'étant pas nécessaire. Il permet également de se passer du carbonyldiimidazole qui a un coût élevé, difficilement adaptable industriellement.

Le procédé selon la présente invention utilise un diarylcarbonate éventuellement substitué qui est un réactif bon marché et ce même pour des molécules dont la cyclisation est difficile à mettre en oeuvre de par leur nature moins nucléophile.

Dans un autre mode de réalisation préféré selon la présente invention, le procédé comprend en outre :
- une étape de mélange à l'état solide dudit composé comprenant un groupe fonctionnel hydroxyaminopyridine de formule (1) éventuellement substitué et dudit composé comprenant un groupe fonctionnel diarylcarbonate (2) éventuellement substitué, et
- une étape de chauffage desdits deux composés en mélange à une température comprise entre 50°C et 170°C, de préférence entre 70°C et 150°C, avantageusement entre 70°C et 120°C, en particulier entre 80°C et 100°C, jusqu'à l'obtention de ladite suspension.

Dans une variante selon la présente invention, le procédé comprend en outre :
- une étape de chauffage dudit composé comprenant un groupe fonctionnel diarylcarbonate (2) éventuellement substitué à un état solide à une température comprise entre 50°C et 170°C, de préférence entre 70°C et 150°C, avantageusement entre 70°C et 120°C, en particulier entre 80°C et 100°C, et
- une étape d'introduction dudit composé comprenant un groupe fonctionnel hydroxyaminopyridine de formule (1) éventuellement substitué à un état solide dans ledit composé comprenant un groupe fonctionnel diarylcarbonate (2) éventuellement substitué à un état fondu, de manière à former ladite suspension.

Avantageusement, le procédé selon la présente invention est caractérisé en ce que ladite étape de chauffage a lieu pendant au moins 1h, avantageusement au moins 2h, en particulier au moins 3h, de préférence au moins 4h, de façon avantageuse au moins 5h, préférentiellement au moins 6h, en particulier au moins 7h, de façon préférée au moins 8h, avantageusement au moins 9h, en particulier au moins 10h, de préférence au moins 11h, de façon avantageuse au moins 12h, préférentiellement au moins 13h, en particulier au moins 14h, de façon préférée au moins 15h, avantageusement au moins 16h, en particulier au moins 17h, de préférence au moins 18h, de façon avantageuse au moins 19h, préférentiellement au moins 20h, en particulier au moins 21h, de façon préférée au moins 22h, avantageusement au moins 23h, de préférence pendant au plus 24h, avantageusement au plus 23h, en particulier au plus 22h, de préférence au plus 21h, de façon avantageuse au plus 20h, préférentiellement au plus 19h, en particulier au plus 18h, de façon préférée au plus 17h, avantageusement au plus 16h, de préférence pendant au plus 15h, avantageusement au plus 14h, en particulier au plus 13h, de préférence au plus 12h, de façon avantageuse au plus 11h, préférentiellement au plus 10h, en particulier au plus 9h, de façon préférée au plus 8h, avantageusement au plus 7h, de préférence pendant au plus 6h, avantageusement au plus 5h, en particulier au plus 4h, de préférence au plus 3h, de façon avantageuse au plus 2h.

Dans un autre mode de réalisation préféré selon la présente invention, le procédé est caractérisé en ce que ledit composé comprenant un groupe fonctionnel hydroxyaminopyridine de formule (1) est la 2-amino-3-hydroxypyridine ou la 3-amino-4-hydroxypyridine ou la 4-amino-3-hydroxypyridine ou la 3-amino-2-hydroxypyridine.

Avantageusement, ledit composé comprenant un groupe fonctionnel hydroxyaminopyridine de formule (1) peut être substitué sur le cycle pyridine respectivement par un ou plusieurs substituants choisis indépendamment l'un de l'autre dans le groupe constitué des radicaux R, OR₁, SR₁, NR₂R₃, NR₄R₅, C(O)R₆ et SO₂R₇ où :
- R représente indépendamment un atome d'hydrogène ou un groupement alkyle, aryle, halogène ou nitro,
- R₁ représente indépendamment un groupement alkyle, aryle ou C(O)R₂,
- R₂ représente indépendamment un groupement alkyle ou aryle,
- R₃ représente indépendamment un groupement alkyle ou aryle,
- R₄ représente un groupement C(O)R₂,
- R₅ représente indépendamment un atome d'hydrogène ou un groupement alkyle ou aryle,
- R₆ représente indépendamment un atome d'hydrogène ou un groupement alkyle, aryle, OR₂ ou NR₂R₃,
- R₇ représente indépendamment un groupement alkyle, aryle, NH₂ ou NR₄R₅.

Avantageusement, le procédé est caractérisé en ce que ledit composé comprenant un groupe fonctionnel hydroxyaminopyridine de formule (1) peut être substitué sur le groupe amino par un groupe alkyle.

Dans une variante selon la présente invention, le procédé est caractérisé en ce que ledit composé comprenant un groupe fonctionnel oxazolopyridinone de formule (3) est substitué sur le cycle pyridine en position 5 et/ou 6 et/ou 7 respectivement par un ou plusieurs substituants choisis indépendamment l'un de l'autre dans le groupe constitué des radicaux R, OR₁, SR₁, NR₂R₃, NR₄R₅, C(O)R₆ et SO₂R₇ où :
- R représente indépendamment un atome d'hydrogène ou un groupement alkyle, aryle, halogène ou nitro,
- R₁ représente indépendamment un groupement alkyle, aryle ou C(O)R₂,
- R₂ représente indépendamment un groupement alkyle ou aryle,
- R₃ représente indépendamment un groupement alkyle ou aryle,
- R₄ représente un groupement C(O)R₂,
- R₅ représente indépendamment un atome d'hydrogène ou un groupement alkyle ou aryle,
- R₆ représente indépendamment un atome d'hydrogène ou un groupement alkyle, aryle, OR₂ ou NR₂R₃,
- R₇ représente indépendamment un groupement alkyle, aryle, NH₂ ou NR₄R₅.

Avantageusement, le procédé est caractérisé en ce que ledit composé comprenant un groupe fonctionnel oxazolopyridinone de formule (3) peut être substitué sur le cycle oxazolidinone en position 3 respectivement par un ou plusieurs substituants choisis indépendamment l'un de l'autre dans le groupe constitué du radical R' où R' représente indépendamment :
- Un groupement alkyle,
- Un groupement halogénométhyle avec l'halogène choisi parmi le chlore (Cl) ou le brome (Br),
- Un groupement 1,2-dihalogénoéthyle avec l'halogène choisi parmi le chlore (Cl) ou le brome (Br),
- Un groupement vinyle,
- Un groupement hydroxyméthyle,
- Un groupement (CH₂)ₙ-X (avec X = P(O)OR" et R" choisi parmi H, alkyle; Br; CN; arylpipérazine),
- Un groupement CH₂SP(O)(OMe)2,

Dans un autre mode de réalisation préféré selon la présente invention, le procédé est caractérisé en ce que ledit composé comprenant un groupe fonctionnel diarylcarbonate de formule (2) comprend, au moins un groupement phényle, de préférence deux groupements phényle.

L'invention concerne également l'utilisation dudit composé préparé selon la présente invention, pour la préparation de l'Azaméthiphos.

En effet, l'Azaméthiphos est une molécule connue pour être insecticide en agissant sur le système nerveux des insectes volants et rampants et des parasites animaux.

L'invention est relative à l'utilisation dudit composé préparé selon la présente invention, pour la préparation d'analogues de la Fosmidomycine.

En effet, la Fosmidomycine est connue pour être un herbicide mais également comme un antibiotique intervenant dans le traitement de la Malaria/Paludisme.

L'invention se rapporte également à l'utilisation dudit composé préparé selon la présente invention, pour la préparation d'analogues de la 3-[(4-aryl-1-piperazinyl)alkyl]oxazolo[4,5-b]pyridin-2-(3H)-ones ou de la 1-[(4-aryl-1-piperazinyl)alkyl]oxazolo[5,4-b]pyridin-2-(1H)-ones ou des composés de type aminohydroxypyridines N-substitués.

En effet, les composés analogues de la 3-[(4-aryl-1-piperazinyl)alkyl]oxazolo[4,5-b]pyridin-2-(3H)-ones, de la 1-[(4-aryl-1-piperazinyl)alkyl]oxazolo[5,4-b]pyridin-2-(1H)-ones ou les composés de type aminohydroxypyridines N-substitués agissent comme composés analgésiques.

Avantageusement, le procédé selon la présente invention est caractérisé en ce qu'il comprend en outre, une deuxième étape de réaction de chloration sur aromatique dudit premier composé comprenant un groupe fonctionnel oxazolopyridinone pour former un deuxième composé comprenant un groupe fonctionnel oxazolopyridinone.

Dans un autre mode de réalisation préféré selon la présente invention, le procédé est caractérisé en ce que ladite deuxième étape de réaction de chloration sur aromatique est réalisée dans un solvant polaire aprotique choisi dans le groupe constitué de l'acétone, de l'acétonitrile, du DMF, du THF, du NMP et de l'acétate d'éthyle, avec du chlore gazeux à froid pour former ledit deuxième composé comprenant un groupe fonctionnel oxazolopyridinone.

Avantageusement, le procédé selon la présente invention est caractérisé en ce qu'il comprend en outre, une troisième étape de réaction dudit deuxième composé comprenant un groupe fonctionnel oxazolopyridinone avec du paraformaldéhyde pour former un troisième composé comprenant un groupe fonctionnel oxazolopyridinone.

De manière particulièrement avantageuse, le procédé selon la présente invention est caractérisé en ce qu'il comprend en outre, une quatrième étape de réaction dudit troisième composé comprenant un groupe fonctionnel oxazolopyridinone avec du chlorure de thionyle pour former un quatrième composé comprenant un groupe fonctionnel oxazolopyridinone, de préférence ledit quatrième composé comprenant un groupe fonctionnel oxazolopyridinone est le 6-chloro-3-(chloromethyl)oxazolo[4,5-b]pyridine-2(3H)-one.

Avantageusement, le procédé selon la présente invention est caractérisé en ce qu'il comprend en outre, une étape d'isolement dudit quatrième composé comprenant un groupe fonctionnel oxazolopyridinone, de préférence par précipitation avec de l'eau, ce qui permet en outre d'isoler le produit dans le but d'augmenter sa pureté.

Dans un autre mode de réalisation préféré selon la présente invention, le procédé est caractérisé en ce qu'il comprend en outre, une cinquième étape de réaction dudit quatrième composé comprenant un groupe fonctionnel oxazolopyridinone avec du O,O'-diméthylphosphorothioate de sodium dans un solvant, de préférence un solvant polaire pour former un cinquième composé comprenant un groupe fonctionnel oxazolopyridinone, comme par exemple l'Azaméthiphos.

Avantageusement, le procédé selon la présente invention est caractérisé en ce qu'il comprend en outre, une étape de précipitation et de purification dudit cinquième composé comprenant un groupe fonctionnel oxazolopyridinone, de préférence par recristallisation, ce qui permet en outre de purifier le produit dans le but d'augmenter sa pureté.

Avantageusement, le procédé selon la présente invention est caractérisé en ce qu'il comprend en outre, au moins une étape de suivi chromatographique, de préférence après chaque étape de réaction.

L'invention se rapporte également à l'utilisation dudit composé préparé selon la présente invention, pour la préparation d'un médicament ou d'un traitement efficace pour traiter les maladies auto immunes, le mélanome, l'arthrite, l'arthrite rhumatoïde, la maladie de Parkinson, l'asthme, la démence, la maladie d'Alzheimer, la leucémie, le diabète, le psoriasis, l'insuffisance cardiaque et les allergies.

D'autres formes de réalisation du procédé de fabrication de dérivés comprenant un groupe caractéristique oxazolopyridinone sont indiquées dans les revendications annexées.

Il est bien entendu que la présente invention n'est en aucune façon limitée aux formes de réalisations décrites ci-dessus et que bien des modifications peuvent y être apportées sans sortir du cadre des revendications annexées.

### Description de l'invention

L'invention va maintenant être décrite de façon plus détaillée, en faisant référence aux réactions suivantes, lesquelles :

La réaction I est une réaction directe selon le procédé de la présente invention entre un composé comprenant un groupe fonctionnel hydroxyaminopyridine de formule (1) éventuellement substitué et, un composé comprenant un groupe fonctionnel diarylcarbonate de formule (2) éventuellement substitué pour former un premier composé de formule (3) éventuellement substitué comprenant un groupe fonctionnel oxazolopyridinone et du phénol de formule (4) tel que décrit ci-dessous :

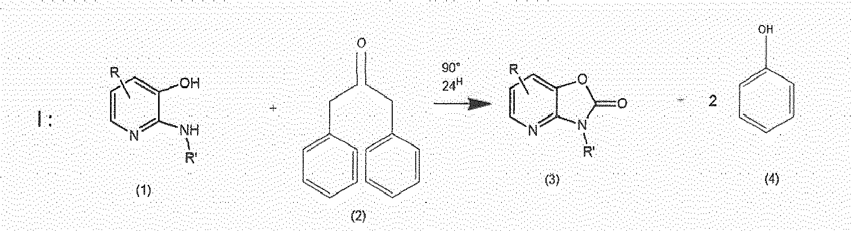

Le premier composé comprenant un groupe fonctionnel oxazolopyridinone de formule (3) peut être substitué sur le cycle pyridine en position 5 et/ou 6 et/ou 7 respectivement par un ou plusieurs substituants choisis indépendamment l'un de l'autre dans le groupe constitué des radicaux R, OR₁, SR₁, NR₂R₃, NR₄R₅, C(O)R₆ et SO₂R₇ où :
- R représente indépendamment un atome d'hydrogène ou un groupement alkyle, aryle, halogène ou nitro,
- R₁ représente indépendamment un groupement alkyle, aryle ou C(O)R₂,
- R₂ représente indépendamment un groupement alkyle ou aryle,
- R₃ représente indépendamment un groupement alkyle ou aryle,
- R₄ représente un groupement C(O)R₂,
- R₅ représente indépendamment un atome d'hydrogène ou un groupement alkyle ou aryle,
- R₆ représente indépendamment un atome d'hydrogène ou un groupement alkyle, aryle, OR₂ ou NR₂R₃,
- R₇ représente indépendamment un groupement alkyle, aryle, NH₂ ou NR₄R₅.

Le premier composé comprenant un groupe fonctionnel oxazolopyridinone de formule (3) peut être substitué sur le cycle oxazolidinone en position 3 respectivement par un ou plusieurs substituants choisis indépendamment l'un de l'autre dans le groupe constitué du radical R' où R' représente indépendamment :
- Un groupement alkyle,
- Un groupement halogénométhyle avec l'halogène choisi parmi le chlore (Cl) ou le brome (Br),
- Un groupement 1,2-dihalogénoéthyle avec l'halogène choisi parmi le chlore (Cl) ou le brome (Br),
- Un groupement vinyle,
- Un groupement hydroxyméthyle,
- Un groupement (CH₂)ₙ-X (avec X = P(O)OR" et R" choisi parmi H,alkyle; Br; CN; arylpipérazine),
- Un groupement CH₂SP(O)(OMe)2,

Le composé comprenant un groupe fonctionnel hydroxyaminopyridine de formule (1) est la 2-amino-3-hydroxypyridine ou la 3-amino-4-hydroxypyridine ou la 4-amino-3-hydroxypyridine ou la 3-amino-2-hydroxypyridine.

En outre, le composé comprenant un groupe fonctionnel diarylcarbonate de formule(2) comprend au moins un groupement phényle, de préférence deux groupements phényle.

### Exemple 1 : Préparation de l'Azaméthiphos

Le composé comprenant un groupe fonctionnel hydroxyaminopyridine de formule (1) à un état solide et le composé comprenant un groupe fonctionnel diarylcarbonate de formule (2) à un état solide vont être chauffés à au moins 85°C avec une étape de mélange du composé comprenant un groupe fonctionnel hydroxyaminopyridine de formule (1) à un état solide dans ledit composé comprenant un groupe fonctionnel diarylcarbonate de formule (2) à un état fondu pour former un premier composé de formule (3) comprenant un groupe fonctionnel oxazolopyridinone et du phénol de formule (4) tel que décrit ci-dessous :
I : 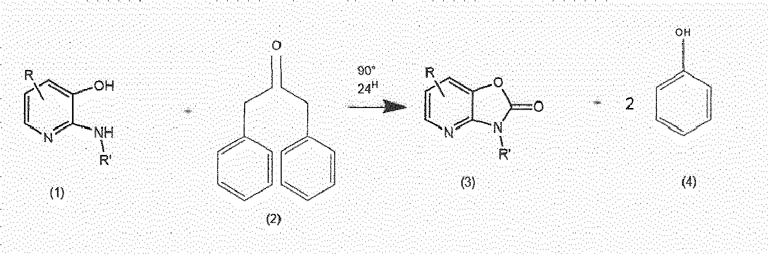 Le premier composé de formule (3) comprenant un groupe fonctionnel oxazolopyridinone peut réagir selon la réaction II de chloration sur aromatique dans un solvant polaire aprotique choisi dans le groupe constitué de l'acétone, de l'acétonitrile, du DMF, du THF, du NMP et de l'acétate d'éthyle, avec du chlore gazeux à froid pour former un deuxième composé de formule (5) comprenant un groupe fonctionnel oxazolopyridinone tel que décrit ci-dessous :
II : 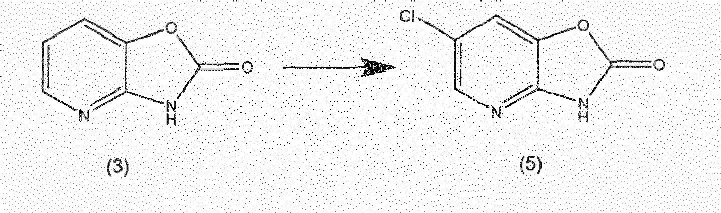 Le deuxième composé de formule (5) comprenant un groupe fonctionnel oxazolopyridinone peut réagir avec du paraformaldéhyde selon la réaction III pour former un troisième composé de formule (6) comprenant un groupe fonctionnel oxazolopyridinone tel que décrit ci-dessous :
III : 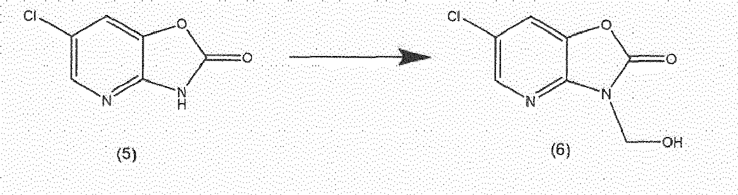 Le troisième composé de formule (6) comprenant un groupe fonctionnel oxazolopyridinone peut réagir avec du chlorure de thionyle avec une température croissante selon la réaction IV pour former un quatrième composé de formule (7) comprenant un groupe fonctionnel oxazolopyridinone tel que décrit ci-dessous :
IV : 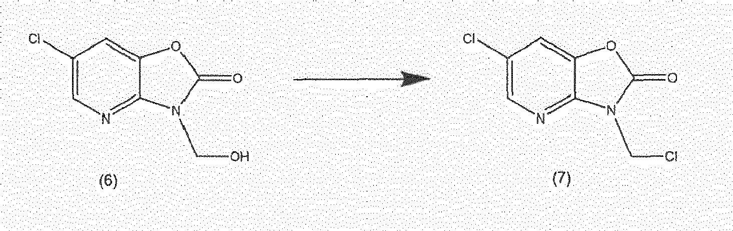 La réaction II, la réaction III et la réaction IV peuvent être réalisées dans un seul réacteur sans isolement des intermédiaires, à savoir, le deuxième composé de formule (5) comprenant un groupe fonctionnel oxazolopyridinone et le troisième composé de formule (6) comprenant un groupe fonctionnel oxazolopyridinone ou réalisées séquentiellement dans des réacteurs séparés, après isolement des intermédiaires.
   Le quatrième composé de formule (7) comprenant un groupe fonctionnel oxazolopyridinone est isolé par précipitation avec de l'eau et peut réagir avec du O,O'-diméthylphosphorothioate de sodium dans un solvant polaire choisi par exemple dans le groupe constitué des cétones aliphatiques primaires telles que l'acétone, la cétone méthyléthylique, des alcanols tels que le méthanol, l'éthanol, l'isopropanol, des esters tels que l'acétate d'éthyle, des nitriles, des acides amides N-alkylés, à froid selon une réaction V pour former un cinquième composé de formule (8) comprenant un groupe fonctionnel oxazolopyridinone, l'Azaméthiphos (8) tel que décrit ci-dessous :
V:

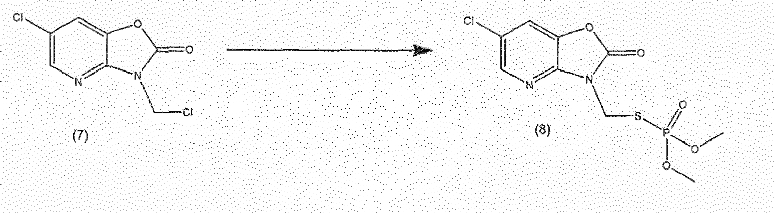

L'Azaméthiphos est précipité et purifié par recristallisation et, l'entièreté du procédé réactionnel est suivi par chromatographie pour s'assurer que chaque étape est complète et déterminer la pureté.

### Exemple 2 : Préparation de composés de type oxazolopyridinone (US3929809)

Le premier composé de formule (3) comprenant un groupe fonctionnel oxazolopyridinone peut réagir selon la réaction VI d'halogénation sur aromatique pour former un sixième composé de formule (9) comprenant un groupe fonctionnel oxazolopyridinone dérivé avec un groupe caractéristique chloro, bromo ou nitro tel que décrit ci-dessous :
VI : 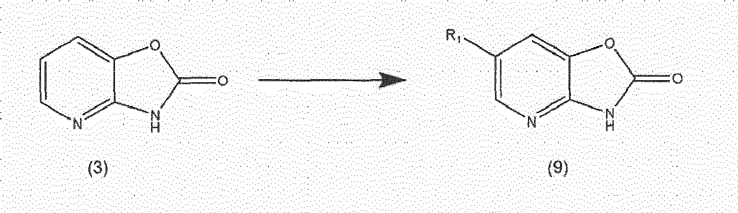 Le sixième composé de formule (9) comprenant un groupe fonctionnel oxazolopyridinone dérivé avec un groupe caractéristique chloro, bromo ou nitro peut réagir selon la réaction VII avec un groupement A choisi dans le groupe des radicaux halogènes méthyles, des vinyles ou des radicaux 1,2-dihalogenoéthyles pour former un septième composé de formule (10) comprenant un groupe fonctionnel oxazolopyridinone tel que décrit ci-dessous :
VII : 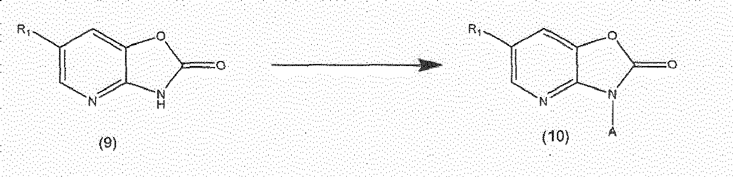 Le septième composé de formule (10) comprenant un groupe fonctionnel oxazolopyridinone peut réagir selon une huitième réaction VIII avec un composé phosphoré, optionnellement en présence d'un agent de liaison acide, ou avec un sel pour former un huitième composé de formule (11) comprenant un groupe fonctionnel oxazolopyridinone tel que décrit ci-dessous :
VIII :

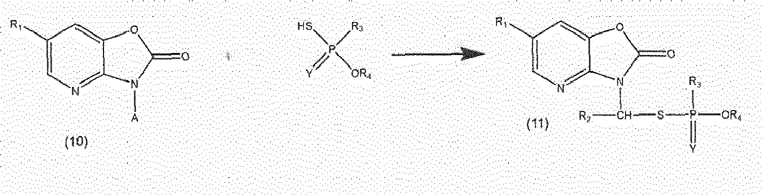

Des exemples du septième composé de formule (10) comprenant un groupe fonctionnel oxazolopyridinone sont les suivants :
- 3-chloromethyl-oxazolo[4,5-b]pyridin-2-(3H)-one
- 3-chloromethyl-oxazolo[4,5-b]pyridin-2(3H)-thione
- 3-chloromethyl-6-chloro-oxazolo[4,5-b]pyridin-2(3H)-one
- 3-bromomethyl-6-chloro-oxazolo[4,5-b]pyridin-2(3H)-one
- 3-chloromethyl-6-bromo-oxazolo[4,5-b]pyridin-2(3H)-one
- 3-chloromethyl-6-nitro-oxazolo[4,5-b]pyridin-2(3H)-one
- 3-vinyl-oxazolo[4,5-b]pyridin-2(3H)-one
- 3-vinyl-oxazolo[4,5-b]pyridin-2(3H)-thione
- 3-vinyl-6-chloro-oxazolo[4,5-b]pyridin-2(3H)-one
- 3-vinyl-6-bromo-oxazolo[4,5-b]pyridin-2(3H)-one
- 3-vinyl-6-nitro-oxazolo[4,5-b]pyridin-2(3H)-one
- 3-(1',2'-dichloroethyl)-oxazolo[4,5-b]pyridin-2-(3H)-one
- 3-(1',2'-dibromoethyl)-oxazolo[4,5-b]pyridin-2(3H)-one
- 3-(1',2'-dichloroethyl)-oxazolo[4,5-b]pyridin-2(3H)-thione
- 3-(1',2'-dibromoethyl)-oxazolo[4,5-b]pyridin-2(3H)-thione
- 3-(1',2'-dichloroethyl)-6-chloro-oxazolo[4,5-b]pyridin-2(3H)-one
- 3-(1',2'-dibromoethyl)-6-chloro-oxazolo[4,5-b]pyridin-2(3H)-one
- 3-(1',2'-dichloroethyl)-6-bromo-oxazolo[4,5-b]pyridin-2(3H)-one
- 3-(1',2'-dibromoethyl)-6-bromo-oxazolo[4,5-b]pyridin-2(3H)-one
- 3-(1',2'-dichloroethyl)-6-nitro-oxazolo[4,5-b]pyridin-2(3H)-one
- 3-(1',2'-dibromoethyl)-6-nitro-oxazolo[4,5-b]pyridin-2(3H)-one

Des exemples du composé phosphoré sont les suivants, et peuvent être fabriqués à partir de procédés connus :
- L'acide O,O-diméthyl-dithiophosphorique
- L'acide O,O-diméthyl-monothiophosphorique
- L'acide O,O-diéthyl-dithiophosphorique
- L'acide O,O-diéthyl-monothiophosphorique
- L'acide O,O-di-isopropyl-dithiophosphorique
- L'acide O-méthyl-O-éthyl-dithiophosphorique
- L'acide O-méthyl-O-isopropyl-dithiophosphorique
- L'acide O-méthyl-O-isopropyl-dithiophosphorique
- L'acide O,O-diallyl-dithiophosphorique
- L'acide O,O-bis-(2-méthoxy-éthyl)-dithiophosphorique
- L'acide O,O-bis-(2-éthoxy-éthyl)-dithiophosphorique
- L'acide O,O-bis-(2-chloroéthyl)-dithiophosphorique
- L'acide O-méthyl-dithio-méthylphosphonique
- L'acide O-éthyl-dithio-méthylphosphonique
- L'acide O-méthyl-dithio-éthylphosphonique
- L'acide O-éthyl-dithio-éthylphosphonique
- L'acide O-méthyl-dithio-phénylphosphonique
- L'acide O-éthyl-dithio-phénylphosphonique
- L'acide amide O-méthyl-thiophosphorique
- L'acide amide O-éthyl-thiophosphorique
- L'acide amide O-isopropyl-thiophosphorique
- L'acide O-méthyl-thiophosphorique-méthyl amide
- L'acide O-éthyl-thiophosphorique-méthyl amide
- L'acide O-méthyl-thiophosphorique-éthyl amide
- L'acide O-éthyl-thiophosphorique-éthyl amide
- L'acide O-méthyl-thiophosphorique-diméthyl amide
- L'acide O-éthyl-thiophosphorique-diméthyl amide
- L'acide O-méthyl-thiophosphorique-diéthyl amide
- L'acide O-éthyl-thiophosphorique-diéthyl amide
- L'acide O-méthyl-dithiophosphorique-diméthyl amide
- L'acide O-éthyl-dithiophosphorique-diméthyl amide
- L'acide O-méthyl-dithiophosphorique-diéthyl amide
- L'acide O-éthyl-dithiophosphorique-diéthyl amide
- L'acide O-isopropyl-dithiophosphorique-diméthyl amide

Le composé phosphoré réagit avec le septième composé de formule (10) comprenant un groupe fonctionnel oxazolopyridinone sans contraintes de températures entre 0°C et 120°C, de préférence entre 10°C et 70°C. Les éventuels solvants polaires sont choisis par exemple dans le groupe constitué des :
- Cétones aliphatiques primaires telles que l'acétone, la cétone méthyléthylique ;
- Alcanols tels que le méthanol, l'éthanol, l'isopropanol ;
- Esters tels que l'acétate d'éthyle ;
- Nitriles;
- Acides amides N-alkylés ;

Les radicaux R1, R2, R3, R4, X et Y mentionnés précédemment sont choisis dans le groupe suivant :
- R1 représente un atome d'hydrogène, halogène ou nitro ;
- R2 représente un atome d'hydrogène ou un groupement méthyl, chlorométhyl ou bromométhyl ;
- R3 représente un groupement alkyl, alkoxy, alkenyloxy, alkynyloxy, alkoxyalkoxy, helogenalkoxy, phenyl, amino, monoalkylamino ou dialkylamino ;
- R4 représente un groupement alkyl, alkenyl, alkynyl, alkoxyalkyl ou helogenalkyl ;
- X représente un atome d'oxygène ou de sulfure ;
- Y représente un atome d'oxygène ou de sulfure ;

### Exemple 3 : Préparation de composés de type analogues de la Fosmidomycine

Le premier composé de formule (3) comprenant un groupe fonctionnel oxazolopyridinone peut réagir selon une neuvième réaction IX avec de l'EtONa dans de l'EtOH pendant 1h à température ambiante puis avec du diéthyl 3-bromopropylphosphonate dans un solvant polaire aprotique choisi dans le groupe constitué de l'acétone, de l'acétonitrile, du DMF, du THF, du NMP et de l'acétate d'éthyle, à reflux pendant une nuit pour former un seizième composé de formule (19) comprenant un groupe fonctionnel oxazolopyridinone tel que mentionné ci-dessous :
IX : 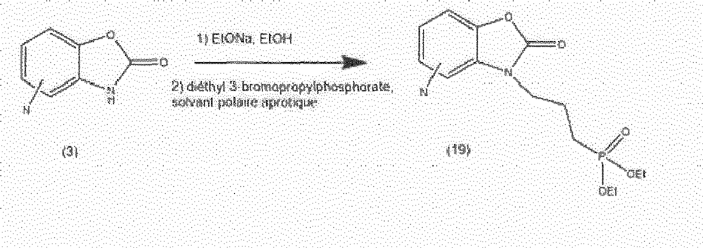 Lequel seizième composé de formule (19) comprenant un groupe fonctionnel oxazolopyridinone peut réagir selon une dixième réaction X avec du (CH₃)₃SiBr dans du CH₂Cl₂, à froid pour former un dix-septième composé de formule (20) comprenant un groupe fonctionnel oxazolopyridinone, analogue de la Fosmidomycine tel que mentionné ci-dessous :
X:

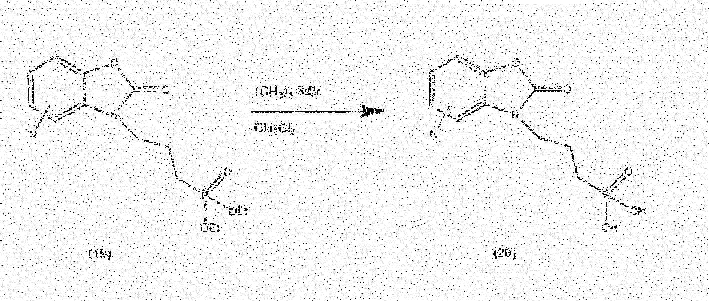

### Exemple 4 : Préparation de composés de type aminohydroxypyridines N-substitués

Le premier composé de formule (3) comprenant un groupe fonctionnel oxazolopyridinone peut réagir selon une onzième réaction XI avec du Br-(CH₂)ₙ-Br en présence de NaH dans du DMF à 110°C pour former un dix-huitième composé de formule (21) comprenant un groupe fonctionnel oxazolopyridinone tel que mentionné ci-dessous :
XI : 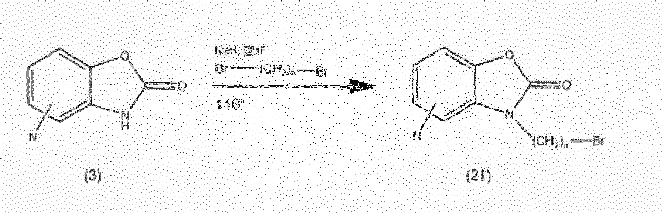 Lequel dix-huitième composé de formule (21) comprenant un groupe fonctionnel oxazolopyridinone peut réagir selon une douzième réaction XII avec du KCN et du KI dans du DMSO pour former un dix-neuvième composé de formule (22) comprenant un groupe fonctionnel oxazolopyridinone tel que mentionné ci-dessous :
XII : 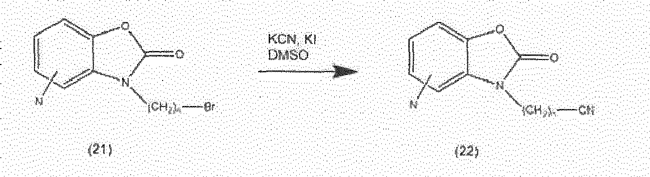 Lequel dix-neuvième composé de formule (22) comprenant un groupe fonctionnel oxazolopyridinone peut réagir selon une treizième réaction XIII avec du NaOH 10% puis du HCl pour former un vingtième composé de formule (23) comprenant un groupe fonctionnel aminohydroxypyridine tel que mentionné ci-dessous :
XIII: 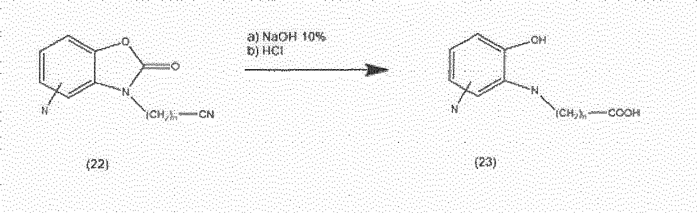 En outre, le dix-huitième composé de formule (21) comprenant un groupe fonctionnel oxazolopyridinone peut réagir selon une quatorzième réaction XIV avec de l'arylpiperazine, en présence du diisopropryléthylamine (iPr)₂NEt dans du CH₃CN pour former un vingt-et-unième composé de formule (24) comprenant un groupe fonctionnel oxazolopyridinone tel que mentionné ci-dessous :
XIV : 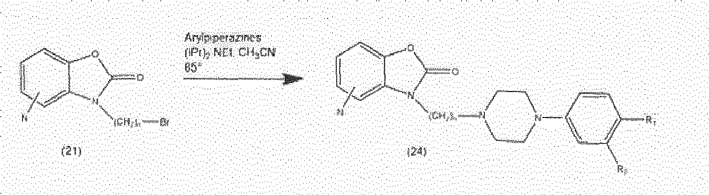 Lequel vingt-et-unième composé de formule (24) comprenant un groupe fonctionnel oxazolopyridinone peut réagir selon une quinzième réaction XV avec du NaOH 10% puis du HCl pour former un vingt-deuxième composé de formule (25) comprenant un groupe fonctionnel aminohydroxypyridine tel que mentionné ci-dessous :
XV:

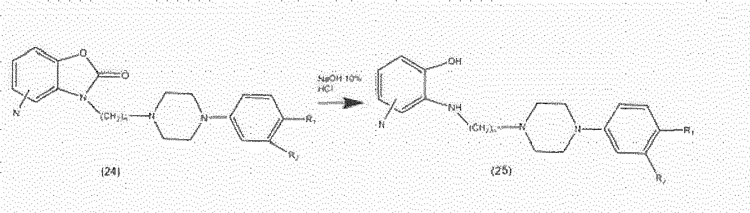

### Exemple 5 : Comparaison du procédé selon l'invention avec et sans solvants et/ou additifs

| Conditions | Conversion (%) (24 h) | Rendement (%) |
|---|---|---|
| Sans solvant, sans additifs | 99.5 | 87.4 à 96.8¹ |
| Na₂CO₃ | 99.5² | - |
| Et₃N | 99.5² | - |
| THF (reflux) | 68 | - |
| EtOAc (reflux) | 57 | - |
| 1,4-dioxane (reflux) | 90 | 56 |

| | | |
|---|---|---|
| ¹ le rendement dépend de la technique d'isolement utilisée. La première technique d'isolement est une extraction du phénol par de l'heptane bouillant pour laquelle il est obtenu 87.4% de rendement. La seconde technique d'isolement est une distillation du phénol et une extraction du phénol restant par de l'heptane bouillant pour laquelle il est obtenu 96.8% de rendement. ² l'effet attendu était la diminutiondu temps de réaction, ce qui n'a pas été observé. | | |

### a) Procédé selon l'invention sans solvant, sans additifs :

On observe que le procédé de réaction I selon l'invention entre un composé comprenant un groupe fonctionnel hydroxyaminopyridine de formule (1) éventuellement substitué et, un composé comprenant un groupe fonctionnel diarylcarbonate de formule (2) éventuellement substitué pour former un premier composé de formule (3) comprenant un groupe fonctionnel oxazolopyridinone et du phénol de formule (4), dans des conditions sans solvants et sans additifs a un taux de conversion de 99.5% après 24h et un rendement de 87.4 à 96.8% selon la technique d'isolation utilisée.

### b) Procédé selon l'invention avec ajout de Na₂CO₃ :

On observe que le procédé selon l'invention avec ajout de Na₂CO₃ à un taux de conversion de 99.5% après 24h alors que l'ajout de Na₂CO₃ devrait entrainer une diminution du temps de réaction par rapport au procédé selon l'invention sans solvant ni additifs.

### c) Procédé selon l'invention avec ajout de Et₃N :

On observe que le procédé selon l'invention avec ajout de Et₃N à un taux de conversion de 99.5% après 24h alors que l'ajout de triéthylamine devrait entrainer une diminution du temps de réaction par rapport au procédé selon l'invention sans solvant ni additifs.

### d) Comparaison du procédé selon l'invention sans solvants, sans additifs avec le procédé selon l'invention avec ajout de THF :

On observe que le procédé selon l'invention avec ajout de tétrahydrofurane comme solvant à un taux de conversion de 68% alors que le procédé selon l'invention sans solvant a un taux de conversion de 99.5%, on observe que l'ajout de solvant entraîne une baisse de la conversion et l'ajout d'étapes d'élimination de matières exogènes.

### e) Comparaison du procédé selon l'invention sans solvants, sans additifs avec le procédé selon l'invention avec ajout de EtOAc :

On observe que le procédé selon l'invention avec ajout d'acétate d'éthyle comme solvant à un taux de conversion de 57% alors que le procédé selon l'invention sans solvant a un taux de conversion de 99.5%, on observe que l'ajout de solvant entraîne une baisse de la conversion et l'ajout d'étapes d'élimination de matières exogènes.

### f) Comparaison du procédé selon l'invention sans solvants, sans additifs avec le procédé selon l'invention avec ajout de 1,4-dioxane :

On observe que le procédé selon l'invention avec ajout de 1,4-dioxane comme solvant à un taux de conversion de 90% et un rendement de 56% alors que le procédé selon l'invention sans solvant a un taux de conversion de 99.5% et un rendement compris entre 87.4% et 96.8%, on observe que l'ajout de solvant entraîne une baisse de la conversion et du rendement et l'ajout d'étapes d'élimination de matières exogènes.

## Revendications

1. Procédé de fabrication de composés comprenant un groupe fonctionnel oxazolopyridinone de formule (3) éventuellement substitué 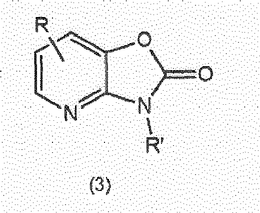 par une réaction entre un composé comprenant un groupe fonctionnel hydroxyaminopyridine de formule (1) éventuellement substitué : 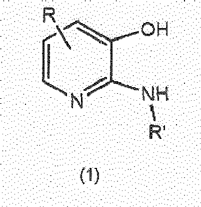 et un composé comprenant un groupe fonctionnel diarylcarbonate de formule (2) éventuellement substitué : 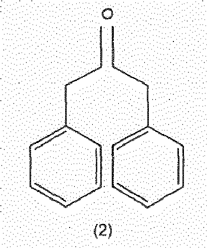 **caractérisé en ce qu'**il comporte une mise en suspension et une réaction dudit composé comprenant un groupe fonctionnel hydroxyaminopyridine de formule (1) dans ledit composé comprenant un groupe fonctionnel diarylcarbonate de formule (2) à un état fondu.

2. Procédé selon la revendication 1, comprenant en outre :
- une étape de mélange à l'état solide dudit composé comprenant un groupe fonctionnel hydroxyaminopyridine de formule (1) éventuellement substitué et dudit composé comprenant un groupe fonctionnel diarylcarbonate (2) éventuellement substitué, et
- une étape de chauffage desdits deux composés en mélange à une température comprise entre 50°C et 170°C, de préférence entre 70°C et 150°C, avantageusement entre 70°C et 120°C, en particulier entre 80°C et 100°C, jusqu'à l'obtention de ladite suspension.

3. Procédé selon la revendication 1, comprenant en outre :
- une étape de chauffage dudit composé comprenant un groupe fonctionnel diarylcarbonate (2) éventuellement substitué à un état solide à une température comprise entre 50°C et 170°C, de préférence entre 70°C et 150°C, avantageusement entre 70°C et 120°C, en particulier entre 80°C et 100°C, et
- une étape d'introduction dudit composé comprenant un groupe fonctionnel hydroxyaminopyridine de formule (1) éventuellement substitué à un état solide dans ledit composé comprenant un groupe fonctionnel diarylcarbonate (2) éventuellement substitué à un état fondu, de manière à former ladite suspension.

4. Procédé selon la revendication 2 ou 3, dans lequel ladite étape de chauffage a lieu entre 1h et 24h.

5. Procédé selon l'une quelconque des revendications 1 à 4, dans lequel ledit composé comprenant un groupe fonctionnel hydroxyaminopyridine de formule (1) est la 2-amino-3-hydroxypyridine ou la 3-amino-4-hydroxypyridine ou la 4-amino-3-hydroxypyridine ou la 3-amino-2-hydroxypyridine.

6. Procédé selon l'une quelconque des revendications 1 à 5, dans lequel ledit composé comprenant un groupe fonctionnel hydroxyaminopyridine de formule (1) peut être substitué sur le cycle pyridine respectivement par un ou plusieurs substituants choisis indépendamment l'un de l'autre dans le groupe constitué des radicaux R, OR₁, SR₁, NR₂R₃, NR₄R₅, C(O)R₆ et SO₂R₇ où :
- R représente indépendamment un atome d'hydrogène ou un groupement alkyle, aryle, halogène ou nitro,
- R₁ représente indépendamment un groupement alkyle, aryle ou C(O)R₂,
- R₂ représente indépendamment un groupement alkyle ou aryle,
- R₃ représente indépendamment un groupement alkyle ou aryle,
- R₄ représente un groupement C(O)R₂,
- R₅ représente indépendamment un atome d'hydrogène ou un groupement alkyle ou aryle,
- R₆ représente indépendamment un atome d'hydrogène ou un groupement alkyle, aryle, OR₂ ou NR₂R₃,
- R₇ représente indépendamment un groupement alkyle, aryle, NH₂ ou NR₄R₅.

7. Procédé selon l'une quelconque des revendications 1 à 6, dans lequel ledit composé comprenant un groupement fonctionnel hydroxyaminopyridine de formule (1) peut être substitué sur le groupe amino par un groupe alkyle.

8. Procédé selon l'une quelconque des revendications 1 à 7, dans lequel ledit composé comprenant un groupe fonctionnel oxazolopyridinone de formule (3) est substitué sur le cycle pyridine en position 5 et/ou 6 et/ou 7 respectivement par un ou plusieurs substituants choisis indépendamment l'un de l'autre dans le groupe constitué des radicaux R, OR₁, SR₁, NR₂R₃, NR₄R₅, C(O)R₆ et SO₂R₇ où :
- R représente indépendamment un atome d'hydrogène ou un groupement alkyle, aryle, halogène ou nitro,
- R₁ représente indépendamment un groupement alkyle, aryle ou C(O)R₂,
- R₂ représente indépendamment un groupement alkyle ou aryle,
- R₃ représente indépendamment un groupement alkyle ou aryle,
- R₄ représente un groupement C(O)R₂,
- R₅ représente indépendamment un atome d'hydrogène ou un groupement alkyle ou aryle,
- R₆ représente indépendamment un atome d'hydrogène ou un groupement alkyle, aryle, OR₂ ou NR₂R₃,
- R₇ représente indépendamment un groupement alkyle, aryle, NH₂ ou NR₄R₅.

9. Procédé selon l'une quelconque des revendications 1 à 8, dans lequel ledit composé comprenant un groupe fonctionnel oxazolopyridinone de formule (3) peut être substitué sur le cycle oxazolidinone en position 3 respectivement par un ou plusieurs substituants choisis indépendamment l'un de l'autre dans le groupe constitué du radical R' où R' représente indépendamment :
- Un groupement alkyle,
- Un groupement halogénométhyle avec l'halogène choisi parmi le chlore (Cl) ou le brome (Br),
- Un groupement 1,2-dihalogénoéthyle avec l'halogène choisi parmi le chlore (Cl) ou le brome (Br),
- Un groupement vinyle,
- Un groupement hydroxyméthyle,
- Un groupement (CH₂)ₙ-X (avec X = P(O)OR" et R" choisi parmi H, alkyle; Br; CN; arylpipérazine),
- Un groupement CH₂SP(O)(OMe)2,

10. Procédé selon l'une quelconque des revendications précédentes, dans lequel ledit composé comprenant un groupe fonctionnel diarylcarbonate de formule (2) comprend, au moins un groupement phényle, de préférence deux groupements phényle.

11. Utilisation dudit composé préparé selon l'une quelconque des revendications 1 à 10, pour la préparation de l'Azaméthiphos.

12. Utilisation dudit composé préparé selon l'une quelconque des revendications 1 à 10, pour la préparation d'analogues de la Fosmidomycine.

13. Utilisation dudit composé préparé selon l'une quelconque des revendications 1 à 10, pour la préparation d'analogues de la 3-[(4-aryl-1-piperazinyl)alkyl]oxazolo[4,5-b]pyridin-2-(3H)-ones ou de la 1-[(4-aryl-1-piperazinyl)alkyl]oxazolo[5,4-b]pyridin-2-(1H)-ones ou des composés de type aminohydroxypyridines N-substitués.

14. Utilisation dudit composé préparé selon l'une quelconque des revendications 1 à 10, pour la préparation d'un médicament ou d'un traitement efficace pour traiter les maladies auto immunes, le mélanome, l'arthrite, l'arthrite rhumatoïde, la maladie de Parkinson, l'asthme, les tumeurs, la démence, la maladie d'Alzheimer, la leucémie, le diabète, le psoriasis, l'insuffisance cardiaque et les allergies.
